# EUROPÄISCHE PATENTSCHRIFT

(11) **EP 2 179 711 B1**
(45) Veröffentlichungstag und Bekanntmachung des Hinweises auf die Patenterteilung: **30.03.2011**
(21) Anmeldenummer: 09012272.2
(22) Anmeldetag: 28.09.2009
(51) Int. Cl.: A61F 5/01, A61F 13/06

(54) **Gelenkbandage**
Joint bandage
Bandage articulé

(30) Priorität: 23.10.2008 DE 102008052860
(43) Veröffentlichungstag der Anmeldung: 28.04.2010
(73) Patentinhaber: Otto Bock HealthCare GmbH, 37115 Duderstadt (DE)
(72) Erfinder: Schlomski, Jens, 37115 Duderstadt (DE); Reinhardt, Holger, 47906 Kempen (DE); Demuth, Peter, 37351 Dingelstädt (DE)
(74) Vertreter: Stornebel, Kai

(56) Entgegenhaltungen:
- WO-A1-94/07443
- DE-A1-102004 040 793

## Beschreibung

Die Erfindung betrifft eine Gelenkbandage mit einem schlauchförmigen Grundkörper aus einem elastischen Textilmaterial mit einem Einsatz in dem Beugebereich, wobei die Elastizität des Einsatzes größer als die des Grundkörpers ist. Die Gelenkbandage ist bevorzugt als Kniebandage einzusetzen, kann jedoch auch beispielsweise als Ellenbogenbandage genutzt werden.

Gelenkbandagen, insbesondere Kniebandagen, sind seit langer Zeit bekannt. Die DE 663 643 betrifft eine Kniebandage, die für Schlottergelenke verwendet werden soll. Die Bandage dient zur Stabilisierung des Kniegelenkes. Bei Kniebandagen tritt das Problem auf, dass bei einer Beugung des Knies die Vorderfläche vergrößert wird, während sich die Kniekehle verkleinert. Dieser Bewegung hat das Material, aus dem eine Kniebandage besteht, zu folgen. Um zu verhindern, dass sich an der Kniekehle das Material der Bandage in Falten legt, ist vorgesehen, dass die Bandage aus zwei verschiedenen Strickwarenteilen zusammengesetzt ist, bei der die Strickware in der einen Richtung auf das Äußerste in die Länge gestreckt worden ist und dadurch in diese Richtung unnachgiebig ist, wobei die Strickware des hinteren Teils der Kniebandage schräg zu den Rändern der Kniebandage verläuft und in einer Richtung nachgiebig und in der anderen Richtung unnachgiebig ist.

Die DE 42 37 398 A1 beschreibt eine Bandage für ein Kniegelenk aus einem elastischen Bandagenstoff in Schlauchform mit einem vorderen Bandagenteil und einen rückwärtigen Bandagenteil und ggf. im rückwärtigen Bandagenteil eingearbeiteten, längsverlaufenden Federstäben. Die Bandage besteht aus einem anatomisch geformten Schlauchkörper mit einem rundumlaufenden Einsatz aus einem hochelastischen Wellengestrick. Im rückwärtigen Bandagenteil ist eine Pelotte aus einem weichen oder weich-elastischen Material eingesetzt, die auf Muskelansätze wirkt.

Die DE 43 22 028 C3 beschreibt eine Gelenkbandage mit einem strumpfförmigen Schlauch aus elastischem, dehnbarem Textilmaterial. Der Schlauch weist im Gelenkbereich einen nahtlos eingearbeiteten Einsatz auf, der in Schlauchlängsrichtung eine größere Dehnbarkeit als in Schlauchumfangsrichtung aufweist und sich maximal über die Hälfte des Schlauchumfanges erstreckt. Der Einsatz besteht aus einem dünneren, elastischeren und eine andere Bindungsstruktur als der Schlauch aufweisendem Material, wobei der Einsatz als in etwa linsenförmige Intarsia-Strickfläche ausgebildet ist, die eine Dehnbarkeit aufweist, die etwa doppelt so groß ist wie die des textilen Schlauchmaterials.

Die DE 297 24 692 U1 beschreibt eine Gelenkbandage mit einem Gestrick als Einsatzteil in einem Gelenkbereich, das Fäden unterschiedlicher Elastizität aufweist. Das Gestrick im Bereich der Gelenkkehle, insbesondere in der Kniekehle, ist in der Bandage nahtlos eingesetzt und weist jeweils zwei Maschen überbrückende Überbrückungsmaschen auf, die in Strickrichtung abwechselnd an beiden Gestrickseiten vorgesehen sind.

Die DE 10 2004 040 793 A1 beschreibt eine elastische Kniegelenkbandage aus einem elastischen Textilmaterial. Um beim Beugen des Knies eine für den Träger der Bandage unangenehme Faltenbildung im Kniegelenk zu vermeiden, wird gegenüber einer Profileinlage im Bereich der Kniekehle ein hochelastischer Einsatz in das Textilmaterial der Bandage eingearbeitet. Dieser Einsatz dehnt sich beim Strecken des Knies und zieht sich bei dessen Beugung wieder zusammen, ohne dass dabei wesentliche Falten entstehen.

Aufgabe der Erfindung ist es, eine verbesserte Gelenkbandage, insbesondere eine verbesserte Kniebandage bereitzustellen, mit der einerseits eine ausreichende Stabilität des Gelenkes und andererseits ein hoher Tragekomfort bereitgestellt werden kann.

Erfindungsgemäß wird diese Aufgabe durch eine Gelenkbandage mit den Merkmalen des Anspruchs 1 gelöst. Vorteilhafte Ausgestaltungen und Weiterbildungen der Erfindung sind in den Unteransprüchen aufgeführt.

Die erfindungsgemäße Gelenkbandage mit einem schlauchförmigen Grundkörper aus einem elastischen Textilmaterial mit einem Einsatz in dem Beugebereich, wobei die Elastizität des Einsatzes größer als die des Grundkörpers ist, sieht vor, dass in dem Einsatz zwei Bereiche unterschiedlicher Elastizität ausgebildet ist, deren Einzelelastizitäten jeweils größer als die des Grundkörpers sind. Durch die Ausbildung des beugeseitigen Einsatzes mit mehreren unterschiedlichen Elastizitäten, die jeweils größer sind als die des Grundkörpers, ist es möglich, eine verbesserte Anlage des Bandagenmaterials im Bereich der Gelenkbeuge zu erreichen, ohne dass eine übermäßige Faltenbildung entsteht. Durch die unterschiedlichen Elastizitäten innerhalb des Einsatzes ist es möglich, eine verbesserte Anpassung der Gewebeeigenschaften im Bereich der Gelenkbeugung bereitzustellen, die an die Eigenschaften der Haut nach Möglichkeit angenähert sind.

Eine Weiterbildung der Erfindung sieht vor, dass in dem Einsatz ein Zentralbereich ausgebildet ist, an den sich zumindest medial und lateral Übergangsbereiche anschließen, die eine größere Elastizität als der Zentralbereich aufweisen. Der Zentralbereich ist dann von Übergangsbereichen mit relativ größerer Elastizität als der Zentralbereich zumindest seitlich umgeben, so dass ein relativ fester Zentralbereich vorhanden ist, der den Einsatz stabilisiert und ein Herauswölben des hochelastischen Gewebes in den Übergangsbereichen verhindert. Der mittelelastische Zentralbereich stabilisiert somit den Einsatz und gibt eine Vorzugsrichtung der Textilbewegung bei der Beugung des Gelenkes vor. Dieser mittelelastische Zentralbereich leitet den gestauchten Gelenkzustand ein, während die hochelastischen Übergangsbereiche, die den Zentralbereich auch vollständig umgeben können, eine Faltenbildung aufgrund der hohen Nachgiebigkeit und Dehnungsfähigkeit verhindern. Der Zentralbereich kann so ausgebildet sein, dass ein definierter Druck auf die Kniekehle ausgeübt werden kann, beispielsweise um eine therapeutische Massagewirkung bereitzustellen.

Der Zentralbereich kann im Wesentlichen sechseckig oder linsenförmig ausgebildet sein, wobei die längste Achse oder die größte Breite des Zentralbereichs in der medial/lateral Ebene liegt. Das Sechseck ist also ein abgeflachtes, verbreitertes Sechseck, bei einer abgerundeten Linsenform liegt lange Achse im Wesentlichen in medial/lateral Ebene des Gelenkes.

Eine Weiterbildung der Erfindung sieht vor, dass die Elastizität des Zentralbereiches in medial/lateral Richtung größer als in proximal/distal Richtung ist, um eine Vorflexion zu erleichtern und eine Vorzugsrichtung der Gewebebewegung in einer Gelenkbeugung vorzugeben.

Die Übergangsbereiche können eine Netzstruktur aufweisen, wodurch ein hoher Durchlüftungsgrad im Bereich der Gelenkbeuge erreicht wird, was einen erhöhten Tragekomfort gewährleistet. Gleichzeitig wird aufgrund der Netzstruktur des hochelastischen Gewebes, Gewirkes oder Gestrickes eine ausreichende Nachgiebigkeit in Bereichen der Bandage bereitgestellt, die in einem hohen Maße gebeugt werden, beispielsweise im Bereich der Sehnenübergänge, wodurch in diesen besonders empfindlichen Bereichen der Gelenkbeuge eine Materialanhäufung und Faltenbildung vermieden werden kann.

Eine Weiterbildung der Erfindung sieht vor, dass die Ränder des Einsatzes gebogen ausgebildet sind, wobei sich die proximalen und distalen Ränder nach innen und die medialen und lateralen Ränder nach außen biegen bzw. gebogen verlaufen. In proximal-distal-Richtung liegt somit eine bikonkave Ausgestaltung vor, während an den seitlichen Rändern eine bikonvexe Ausgestaltung der Ränder vorhanden ist. Eine solche Ausgestaltung des Einsatzes ergibt sich insbesondere im angelegten Zustand der Bandage, kann jedoch auch im flach ausgestreckten Zustand erreicht werden. Durch die Ausgestaltung der proximalen und distalen Ränder mit einer Beugung nach innen wird eine der natürlichen Gelenkbeuge nachempfundene Elastizität und Ausgestaltung der im Vergleich zu dem Grundkörper höher elastischen Bereiche bereitgestellt.

Der Einsatz kann in dem Grundkörper eingearbeitet sein, insbesondere eingestrickt. Dadurch werden störende Nähte im Übergangsbereich vom Grundkörper zum Einsatz in der Gelenkbeuge vermieden.

Die Gelenkbandage ist bevorzugt vorflektiert, um den Tragekomfort zu erhöhen. Der schlauchartige Grundkörper kann von seinen Enden aus gesehen jeweils konisch zusammenlaufen, um eine an den jeweiligen Gliedmaß angenäherte Formgebung zu erhalten, die den Tragekomfort erhöht. Ebenfalls können Versteifungsstreben vorgesehen sein, bevorzugt im medialen und lateralen Bereich, um eine erhöhte Gelenkstabilität durch die Bandage erzielen zu können. Pelotten, beispielsweise Ringpelotten zur Aufnahme der Kniescheibe oder Druckpelotten für den Ellenbogen können an der Streckseite beziehungsweise medial-lateral für eine Ellenbogenbandage vorgesehen sein.

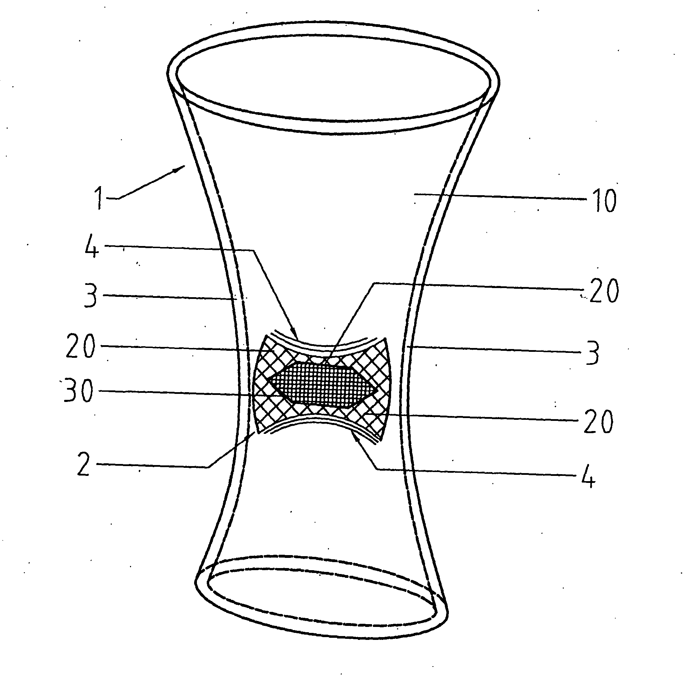

Nachfolgend wird ein Ausführungsbeispiel der Erfindung anhand der beigefügten Figur näher erläutert. Die Figur zeigt eine schematische, perspektivische Ansicht einer Gelenkbandage in Rückansicht.

In der einzigen Figur ist eine Gelenkbandage 1 in einer Rückansicht dargestellt. Die Gelenkbandage 1 weist einen Grundkörper 10 auf, der aus einem schlauchartigen, elastischen Textil besteht. Der Grundkörper 10 kann sowohl in Längsrichtung als auch in Umfangsrichtung gedehnt werden, wobei die Elastizität des Grundkörpers 10 dergestalt ausgewählt ist, dass einerseits eine ausreichende Stabilisierung des Gelenkes im angelegten Zustand der Gelenkbandage 1 erreicht wird, darüber hinaus die Bandage 1 sicher an den Gliedmaßen festgelegt wird und weiterhin ein ausreichender Tragekomfort gegeben ist, ohne das von der Bandage 1 umschlossene Gewebe zu stark zu komprimieren.

An den Seiten der Gelenkbandage 1, also im Medial-Lateral-Bereich, sind Versteifungsstäbe oder Federstäbe 3 eingearbeitet, die einerseits die Gelenkbandage 1 in ihrer Längserstreckung stabilisieren und andererseits das bandagierte Gelenk unterstützen. Die Versteifungsstäbe 3 sind hinreichend biegsam, damit die Beugung in dem Gelenk möglichst ungehindert stattfinden kann.

Der Grundkörper 10 kann eine konische Erweiterung zu zumindest einer Öffnung hin aufweisen, für den Fall des Einsatzes als eine Kniegelenkbandage bietet sich eine konisch erweiternde Öffnung des am Oberschenkel anliegenden Teils der Bandage 1 an, um den Tragekomfort zu erhöhen. Der unterschenkelseitige Teil, also die untere Öffnung des schlauchförmigen Grundkörpers 10, kann zylindrisch oder sich ebenfalls in Richtung auf die Öffnung konisch erweiternd ausgebildet sein. Ebenfalls ist es möglich, dass die Gelenkbandage 1 vorflektiert ist, also eine Vorformung in Beugerichtung aufweist.

Im Beugebereich der Gelenkbandage 1, bei einer Kniegelenkbandage im Bereich der Kniekehle ist ein elastischer Einsatz 2 vorgesehen, der einen Zentralbereich 30 aufweist.

Der Zentralbereich 30 weist eine höhere Elastizität als der Grundkörper 10 auf und ist im dargestellten Ausführungsbeispiel sechseckig ausgebildet. Die Elastizität des Zentralbereiches 30 kann in medial-lateral Richtung größer als in proximal-distal Richtung ausgebildet sein, so dass sich die Gelenkbandage 1 im Beugebereich in Umfangsrichtung leichter ausdehnen kann als in Längsrichtung. Der Zentralbereich 30 ist von Übergangsbereichen 20 umgeben, die im dargestellten Ausführungsbeispiel den Zentralbereich 30 vollständig umgeben. Die Übergangsbereiche 20 sind aus einem netzartigen, hochelastischen Textil, beispielsweise Gestrick ausgebildet, dessen Elastizität höher als die des Zentralbereiches 30 ist. Auch für die Übergangsbereiche 20 können unterschiedliche Elastizitäten in unterschiedlichen Streckrichtungen vorgesehen sein, es besteht jedoch auch die Möglichkeit, gleiche Elastizitäten sowohl in medial/lateral Richtung als auch in proximal/distal Richtung auszubilden.

Als Elastizität wird dabei das Maß verstanden, um das das Textil gelängt werden kann, ohne zerstört zu werden. Kann ein Textilstück durch Ziehen in einer Richtung in seiner Ausdehnung verdoppelt werden, liegt eine Elastizität von 100% vor. Die Elastizität in Längserstreckung des Grundkörpers der Bandage, also in proximaldistal-Richtung, liegt beispielsweise zwischen 80% und 130%, die des Übergangsbereiches 20 zwischen 120% und 180% und die des Zentralbereiches 30 zwischen 100% und 140%. In Umfangsrichtung, also in medial-lateral-Richtung können Elastizitäten zwischen 180% und 220% vorgesehen sein, wobei bevorzugt die Elastizität in Umfangsrichtung größer als die in Längsrichtung ausgebildet ist. Die Elastizität insbesondere im Bereich des Übergangsbereiches20 und des Zentralbereiches 30 in Umfangsrichtung wird durch einen Verzicht aud den Schussfaden erzielt.

Der sechseckige Zentralbereich 30 weist eine abgeflachte Form auf, wobei die Verbindungslinie der beiden einander gegenüberliegenden, am weitesten voneinander entfernten Ecken im Wesentlichen mit der Beugeachse, die angenähert in medial/lateral Richtung orientiert ist, zusammenfällt. Alternativ zu einer sechseckigen Ausgestaltung des Zentralbereiches 30 kann dieser auch linsenförmig ausgebildet sein.

Der gesamte Einsatz 2 weist in medial/lateral Richtung eine bikonvexe Auswölbung nach außen auf, während in proximal/distal Richtung eine bikonkave Einwölbung in Richtung auf den Zentralbereich 30 vorliegt, wodurch die Vorflexion erleichtert wird und verhindert wird, dass sich im gebeugten Zustand Falten bilden. Aufgrund der hohen Elastizitäten im Bereich des Einsatzes 2 und der Vorflexion der Bandage 1 ist es möglich, dass bei einer Beugung des Gelenkes bei angelegter Bandage 1 keine Materialausbeulung in der Gelenkbeuge entsteht, wodurch eine Belastung auf die Sehnen oder Blutgefäße vermieden oder zumindest verringert werden kann.

Der Einsatz 2 kann in dem Grundkörper 10 eingearbeitet sein, insbesondere eingestrickt auf andere Weise bei der Herstellung des Grundkörpers 10 eingearbeitet sein. Der Einsatz 2 simuliert das Verhalten der Haut in einer Gelenkbeuge, wobei der Zentralbereich 30 mit der Elastizität größer als der des Grundkörpers 10 und kleiner als der der Übergangsbereiche 20 eine Einleitung des gestauchten Gelenkzustandes bei dem Beginn der Beugung gewährleistet.

Die Übergangsbereiche 20 vermeiden eine Faltenbildung und bewirken eine enge Anlage des Zentralbereiches 30 in der Gelenkbeuge, wodurch gleichzeitig auch ein therapeutischer Druck auf das Gewebe in der Gelenkbeuge ausgeübt werden kann.

In Transferzonen 4 distal und proximal des Einsatzes 2 ist vorgesehen, dass die Elastizität des Grundkörpers 10 schrittweise zunimmt, beispielsweise indem nur jede zweite oder dritte Reihe des Gestricks mit einem Schussfaden versehen ist. Ebenfalls können Transferzonen auch medial und/oder lateral des Einstzes 2 ausgebildet werden, um einen sanften Übergang von dem relativ festen Grundkörper 10 zu dem hochelastischen Übergangsbereich 20 und dem im Vergleich zu dem Grundkörper 10 höherelastischen Zentralbereich 30 bereitzustellen.

## Patentansprüche

1. Gelenkbandage mit einem schlauchförmigen Grundkörper aus einem elastischen Textilmaterial mit einem Einsatz in dem Beugebereich, wobei die Elastizität des Einsatzes größer als die des Grundkörpers ist, **dadurch gekennzeichnet, dass** in dem Einsatz (2) zwei Bereiche (20, 30) unterschiedlicher Elastizität ausgebildet sind, deren Einzelelastizitäten größer als die des Grundkörpers (10) sind.

2. Gelenkbandage nach Anspruch 1, **dadurch gekennzeichnet, dass** in dem Einsatz (2) ein Zentralbereich (30) ausgebildet ist, an den sich zumindest medial und lateral Übergangsbereiche (20) anschließen, die eine größere Elastizität als der Zentralbereich (30) aufweisen.

3. Gelenkbandage nach Anspruch 2, **dadurch gekennzeichnet, dass** der Zentralbereich (30) im Wesentlichen sechseckig oder linsenförmig ausgebildet ist.

4. Gelenkbandage nach Anspruch 2 oder 3, **dadurch gekennzeichnet, dass** die Elastizität des Zentralbereiches (30) in medial/lateral Richtung größer als in proximal/distal Richtung ist.

5. Gelenkbandage nach einem der Ansprüche 2 bis 4, **dadurch gekennzeichnet, dass** die längste Achse des Zentralbereiches (30) in der medial/lateral Ebene liegt.

6. Gelenkbandage nach einem der Ansprüche 2 bis 5, **dadurch gekennzeichnet, dass** die Übergangsbereiche (20) eine Netzstruktur aufweisen.

7. Gelenkbandage nach einem der Ansprüche 2 bis 6, **dadurch gekennzeichnet, dass** die Elastizität der Übergangsbereiche (20) in medial/lateral Richtung größer als in proximal/distal Richtung ist.

8. Gelenkbandage nach einem Ansprüche 2 bis 7, **dadurch gekennzeichnet, dass** der Zentralbereich (30) in den Übergangsbereichen (20) eingearbeitet ist.

9. Gelenkbandage nach einem der voranstehenden Ansprüche, **dadurch gekennzeichnet, dass** die Ränder des Einsatzes (2) gebogen ausgebildet sind, wobei die Ränder in proximal/distal Erstreckung nach innen und die Ränder in medial/lateral Erstreckung nach außen gebogen sind.

10. Gelenkbandage nach einem der voranstehenden Ansprüche, **dadurch**
**gekennzeichnet, dass** der Einsatz (2) in dem Grundkörper (10) eingearbeitet ist.

11. Gelenkbandage nach einem der voranstehenden Ansprüche, **dadurch**
**gekennzeichnet, dass** die Gelenkbandage (1) vorflektiert ist.

12. Gelenkbandage nach einem der voranstehenden Ansprüche, **dadurch**
**gekennzeichnet, dass** die Gelenkbandage (1) als ein Gestrick ausgebildet ist.

## Claims

1. A joint bandage having a tubular base body of an elastic textile material with an insert in the area of the bend in the joint, such that the elasticity of the insert is greater than that of the base body, **characterized in that** two areas (20, 30) of differing elasticity are formed in the insert (2), their individual elasticities being greater than that of the base body (10).

2. The joint bandage according to claim 1, **characterized in that** a central area (30) is formed in the insert (2) and is connected to transitional areas (20) at least medially and laterally, each of these transitional areas having a greater elasticity than the central area (30).

3. The joint bandage according to claim 2, **characterized in that** the central area (30) is designed to be essentially hexagonal or lenticular.

4. The joint bandage according to claim 2 or 3, **characterized in that** the elasticity of the central area (30) is greater in the mediolateral direction than in the proximal-distal direction.

5. The joint bandage according to any one of claims 2 to 4, **characterized in that** the longest axis of the central area (30) lies in the mediolateral plane.

6. The joint bandage according to any one of claims 2 to 5, **characterized in that** the transitional areas (20) have a mesh structure.

7. The joint bandage according to any one of claims 2 to 6, **characterized in that** the elasticity of the transitional area (20) in the mediolateral direction is greater than that in the proximal-distal direction.

8. The joint bandage according to any one of claims 2 to 7, **characterized in that** the central area (30) is incorporated into the transitional areas (20).

9. The joint bandage according to any one of the preceding claims, **characterized in that** the edges of the insert (2) are designed with a curvature, such that the edges have an inward curvature in the proximal-distal extent and an outward curvature in the mediolateral extent.

10. The joint bandage according to any one of the preceding claims, **characterized in that** the insert (2) is incorporated into the base body (10).

11. The joint bandage according to any one of the preceding claims, **characterized in that** the joint bandage (1) is preflexed.

12. The joint bandage according to any one of the preceding claims, **characterized in that** the joint bandage (1) is designed as a knit fabric.

## Revendications

1. Bandage articulaire comprenant un corps de base en forme de gaine manchon tuyau en un matériau textile élastique ayant un insert dans la région de flexion, l'élasticité de l'insert étant supérieure à celle du corps de base, **caractérisé en ce que** dans l'insert (2) sont formées deux régions (20, 30) d'élasticités différentes dont les élasticités sont supérieures à celle du corps de base (10).

2. Bandage articulaire selon la revendication 1, **caractérisé en ce qu'**une région centrale (30) est formée dans l'insert (2), à laquelle se raccordent au moins des régions de transition (20) médianes et latérales qui présentent une élasticité supérieure à la région centrale (30).

3. Bandage articulaire selon la revendication 2, **caractérisé en ce que** la région centrale (30) est formée sensiblement hexagonale ou en forme de lentille.

4. Bandage articulaire selon la revendication 2 ou 3, **caractérisé en ce que** l'élasticité de la région centrale (30) est supérieure dans la direction milieu/latéral que dans la direction proximal/distal.

5. Bandage articulaire selon l'une quelconque des revendications 2 à 4, **caractérisé en ce que** l'axe le plus long de la région centrale (30) se trouve dans le plan milieu/latéral.

6. Bandage articulaire selon l'une quelconque des revendications 2 à 5, **caractérisé en ce que** les régions de transition (20) présentent une structure réticulaire.

7. Bandage articulaire selon l'une quelconque des revendications 2 à 6, **caractérisé en ce que** l'élasticité des régions de transition (20) est supérieure dans la direction milieu/latéral que dans la direction proximal/distal.

8. Bandage articulaire selon l'une quelconque des revendications 2 à 7, **caractérisé en ce que** la région centrale (30) est incorporée dans les
régions de transition (20).

9. Bandage articulaire selon l'une quelconque des revendications précédentes, **caractérisé en ce que** les bords de l'insert (2) sont formés courbés, les bords étant courbés vers l'intérieur dans l'étendue proximal/distal et vers l'extérieur dans l'étendue milieu/latéral.

10. Bandage articulaire selon l'une quelconque des revendications précédentes, **caractérisé en ce que** l'insert (2) est incorporé dans le corps de base (10).

11. Bandage articulaire selon l'une quelconque des revendications précédentes, **caractérisé en ce que** le bandage articulaire (1) est fléchi à l'avance.

12. Bandage articulaire selon l'une quelconque des revendications précédentes, **caractérisé en ce que** le bandage articulaire est formé par un tricotage.
